# EUROPEAN PATENT APPLICATION

(11) **EP 1 964 516 A1**
(43) Date of publication of application: **03.09.2008**
(21) Application number: 06834345.8
(22) Date of filing: 08.12.2006
(51) Int. Cl.: A61B 5/151, A61B 5/15

(54) **INSERTION NEEDLE AND LANCET WITH THE SAME**

(30) Priority: 08.12.2005 JP 2005355351
(71) Applicant: Arkray, Inc., Kyoto-shi, Kyoto 601-8045 (JP)
(72) Inventor: SAKATA, Tetsuya, Minami-ku, Kyoto-shi, Kyoto 6018045 (JP); KOMURO, Hidefumi, Minami-ku, Kyoto-shi, Kyoto 6018045 (JP)
(74) Representative: HOFFMANN EITLE
(86) International application number: PCT/JP2006/324590
(87) International publication number: WO 2007/066772

(57) **Abstract**

The present invention relates to a puncture needle (11) formed with a first blade surface (12A), a second blade surface (12B), and a third blade surface (12C). The first blade surface (12A) has a site most distant from a needlepoint formed within a range of 1.5 and 2.5 mm as a distance (D1) in an axial direction (L1) from the needlepoint, and the second and third blade surfaces (12B, 12C) have a site most distant from the needlepoint formed in a range of 0.4 to 0.68 mm as a distance (D2) in the axial direction (L1) from the needlepoint. A ratio (D2/D1) of the distance (D2) with respect to the distance (D1) is preferably set in a range of 0.22 to 0.38.

## Description

### TECHNICAL FIELD

The present invention relates to a puncture needle used to collect body fluid such as blood and interstitial fluid by incising skin and the like, and a lancet.

### BACKGROUND ART

A method of measuring blood glucose level includes a method of using a biosensor from prior art. As one example, there is known a method in which a user attaches a biosensor to a portable blood glucose level measuring device that can be carried around, and drops the blood collected from skin to the biosensor to automatically measure the blood glucose level in the blood glucose level measuring device (refer to, e.g., Patent Document 1).

A lacing device used by attaching a lancet is used to collect blood from the skin (e.g., Patent Document 2). As in a schematic configuration shown in Fig. 1, a lancing device 2 includes a lancet holder 20 for holding a lancet 1. The lancet holder 20 can be moved towards the skin Sk by a snapping force of a coil spring 24, and is moved towards the skin Sk while holding the lancet 1 so that a puncture needle 11. of the lancet 1 is inserted to the skin Sk.

A lancing device having a configuration in which a puncturing depth (puncture depth) of the puncture needle to the skin is adjustable is also known (refer to, e.g., patent document 3). If the puncture depth is large, great amount of bleeding from the skin can be ensured but the pain (boring pain) in time of insertion becomes greater. If the puncture depth is small, the boring pain becomes small, but the bleeding amount from the skin becomes small. The bleeding amount and the boring pain depend not only on the puncture depth, but also on the thickness of the puncture needle, where the bleeding amount becomes greater and the boring pain becomes greater with a thick puncture needle than with a thin puncture needle.

Considering the load of the person from whom the blood is to be taken, necessary amount of blood needs to be reliably ensured with one insertion and repeating the insertion due to lack of blood needs to be avoided, and furthermore, the boring pain needs to be alleviated as much as possible. However, the bleeding amount and the boring pain are in a trade-off relationship, and thus it is difficult to bleed the necessary amount of blood while alleviating the boring pain.

Patent Document 1: Japanese Unexamined Patent Publication No. 2003-156469
Patent Document 2: Japanese Unexamined Patent Publication No. 07-275223
Patent Document 3: Japanese Unexamined Patent Publication No. 10-508527

### DISCLOSURE OF THE INVENTION

### PROBLEMS TO BE SOLVED BY THE INVENTION

It is an object of the present invention to provide a puncture needle capable of bleeding the necessary amount of blood while alleviating the boring pain, and a lancet equipped with the same.

### MEANS FOR SOLVING THE PROBLEMS

A puncture needle provided by a first aspect of the present invention is a puncture needle having a blade arranged at an end; wherein the blade includes a first blade surface, a second blade surface, and a third blade surface; the first blade surface is arranged at a position evacuated from a needlepoint than the second and the third blade surfaces as a whole, and has a site most distant from the needlepoint formed within a range of 1.5 to 2.5 mm as a distance D1 in an axial direction from the needlepoint; and the second and third blade surfaces define the needlepoint, and a site most distant from the needlepoint is formed within a range of 0.4 to 0.68 mm as a distance D2 in the axial direction from the needlepoint.

A ratio (D2/D1) of the distance D2 with respect to the distance D1 is preferably set in a range of 0.22 to 0.38.

(D1/D2/R) is preferably set in a range of 8.5 to 31.3 where R is an outer diameter of a portion other than the blade.

A cross-section at the site most distant from the needlepoint of the second and the third blade surfaces preferably has a ratio (D3/D4) of a dimension D3 in a first radial direction along a normal line of the first blade surface and a dimension D4 in a second radial direction orthogonal to the first radial direction set in a range of 0.29 to 0.50, and the ratio (D3/D4) is more preferably set in a range of 0.33 to 0.50.

A puncture needle provided by a second aspect of the present invention relates to a puncture needle having a blade arranged at an end; wherein the blade includes a first blade surface, a second blade surface, and a third blade surface; the first blade surface is arranged at a position evacuated from a needlepoint than the second and the third blade surfaces as a whole; the second and third blade surfaces define the needlepoint; and a ratio (D2/D1) of a distance D2 in an axial direction from the needlepoint of a site most distant from the needlepoint in the second and the third blade surfaces with respect to a distance D1 in the axial direction from the needlepoint of a site most distant from the needlepoint in the first blade surface is set in a range of 0.22 to 0.38.

(D1/D2/R) is preferably set in a range of 8.5 to 31.3 where R is an outer diameter of a portion other than the blade.

A cross-section at the site most distant from the needlepoint of the second and the third blade surfaces preferably has a ratio (D3/D4) of a dimension D3 in a first radial direction along a normal line of the first blade surface and a dimension D4 in a second radial direction orthogonal to the first radial direction set in a range of 0.29 to 0.50, and the ratio (D3/D4) is more preferably set in a range of 0.33 to 0.50.

A puncture needle provided by a third aspect of the present invention relates to a puncture needle having a blade arranged at an end; wherein the blade includes a first blade surface, a second blade surface, and a third blade surface; the first blade surface is arranged at a position evacuated from a needlepoint than the second and the third blade surfaces as a whole; the second and third blade surfaces define the needlepoint; and (D1/D2/R) is set in a range of 8.5 to 31.3 where D1 is a distance in an axial direction from the needlepoint of a site most distant from the needlepoint in the first blade surface, D2 is a distance in the axial direction from the needlepoint of a site most distant from the needlepoint in the second and the third blade surfaces, and R is an outer diameter of a portion other than the blade.

A cross-section at the site most distant from the needlepoint of the second and the third blade surfaces preferably has a ratio (D3/D4) of a dimension D3 in a normal line direction of the first blade surface and a dimension D4 in a direction orthogonal to the normal line direction set in a range of 0.29 to 0.50, and the ratio (D3/D4) is more preferably set in a range of 0.33 to 0.50.

A puncture needle provided by a fourth aspect of the present invention relates to a puncture needle having a blade arranged at an end; wherein the blade includes a first blade surface, a second blade surface, and a third blade surface; the first blade surface is arranged at a position evacuated from a needlepoint than the second and the third blade surfaces as a whole; the second and third blade surfaces define the needlepoint; and a cross-section at a site most distant from the needlepoint in the second and the third blade surfaces has a ratio (D3/D4) of a dimension D3 in a first radial direction along a normal line of the first blade surface and a dimension D4 in a second radial direction orthogonal to the first radial direction set in a range of 0.29 to 0.50.

The ratio (D3/D4) is preferably set in a range of 0.33 to 0.50.

For instance, the first blade surface has an intersecting angle θ1 with respect to the axial direction of the puncture needle of 7 to 20 degrees.

For instance, the second and the third blade surfaces are formed symmetric or substantially symmetric to each other with respect to an axis of symmetry extend in a radial direction along a normal line of the first blade surface when viewed in the axial direction. In this case, a sandwiching angle θ2 between the second blade surface and the third blade surface is set to 120 to 170 degrees etc.

The outer diameter R is 0.2 to 0.4 mm etc.

The ratio (D1/R) of the distance D1 with respect to the outer diameter R is 3.75 to 12.50 etc., and the ratio (D2/R) of the distance D2 with respect to the outer diameter R is 1.0 to 3.5 etc.

In a fifth aspect of the present invention, a lancet including a puncture needle, and a body for holding the puncture needle, wherein the puncture needle is as described in any one of first to fourth aspects of the present invention is provided.

### BRIEF DESCRIPTION OF THE DRAWINGS

Fig. 1 is a cross-sectional view showing a state in which a lancet is attached to a lancing device.
Fig. 2A is a perspective view showing one example of the lancet according to the present invention, and Fig. 2B is a cross-sectional view taken along line IIB-IIB of Fig. 2A.
Fig. 3A is a front view showing an end of a puncture needle according to the present invention, Fig. 3B is a side view thereof, Fig. 3C is a cross-sectional view taken along line IIIC-IIIC of Fig. 3A, Fig. 3D is a cross-sectional view taken along line IIID-IIID of Fig. 3A.
Fig. 4A is a front view showing an end of a conventional puncture needle, Fig. 4B is a side view thereof, Fig. 4C is a cross-sectional view taken along line IVC-IVC of Fig. 4A, Fig. 4D is a cross-sectional view taken along line IVD-IVD of Fig. 4A.
Fig. 5 is a view for comparing the cross-sectional shapes of an end of the puncture needle according to the present invention and an end of the conventional puncture needle.
Fig. 6 is a graph showing change in cross-sectional area at the end of the puncture needle used in example 2.
Fig. 7 is a graph showing a relationship of an puncture depth and a thrust resistance in example 3.
Fig. 8 is a circle graph showing results of a monitor test in example 4.

### EXPLANATION OF SYMBOLS

- 1: Lancet
- 10: Body (of lancet)
- 11: Puncture needle (of lancet)
- 12: Blade (of puncture needle)
- 12A: First blade surface (of puncture needle)
- 12B: Second blade surface (of puncture needle)
- 12C: Third blade surface (of puncture needle)
- R1: First radial direction
- R2: Second radial direction
- L1: Axial direction

### BEST MODE FOR CARRYING OUT THE INVENTION

The present invention will be described with reference to the drawings.

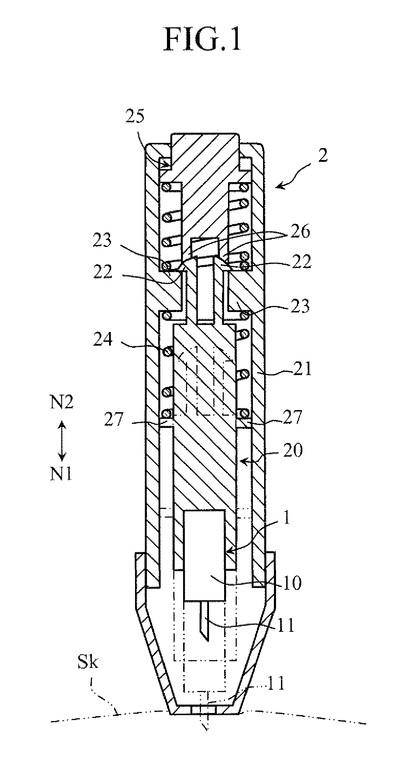

As shown in Fig. 1, a lancet 1 according to the present invention is used by being attached to a lancing device 2. The lancing device 2 shown in Fig. 1 includes a lancet holder 20 for holding the lancet 1.

The lancet holder 20 is accommodated so as to be movable in N1, N2 directions in the interior of a housing 21, and is configured to be biased towards the N1 direction by a coil spring 24 by engaging a pair of latches 22 to a convex part 23 of the housing 21.

The engagement state of the latch 22 is released by a pushing operation of an operation cap 25. That is, the operation cap 25 is slidable in the N1, N2 directions with respect to the housing 21, and a pair of projections 26 interferes with the pair of latches 22 when moved in the N1 direction. Each of the pair of latches 22 is then displaced towards the inner side, and the engagement state of the latch 22 is released.

The coil spring 24 is arranged between the convex part 23 of the housing 21 and a convex part 27 of the lancet holder 20, and is compressed with the latch 22 of the lancet holder 20 engaged to the convex part 23 of the housing 21. Thus, when the latch 22 is engaged to the convex part 23, the lancet holder 20 is biased in the N1 direction by the coil spring 24. When the engagement of the latch 22 to the convex part 23 is released, the lancet holder 20 moves towards the N1 direction by the snapping force of the coil spring 24, and the puncture needle 11 of the lancet 1 punctures the skin Sk.

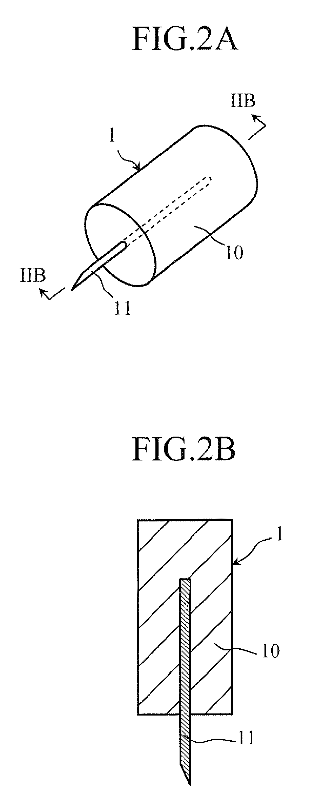

As shown in Figs. 2A and 2B, the lancet 1 has a mode in which the puncture needle 11 is projecting out from a distal end face of a body 10.

The body 10 is the portion to be held at the lancet holder 20 of the lancing device 2, and holds the puncture needle 11. The body 10 is formed to a cylindrical shape by synthetic resin and the like.

The puncture needle 11 is inserted to the skin Sk by means of the lancing device 2 (see Fig. 1), and is insert molded, for example, with respect to the body 10.

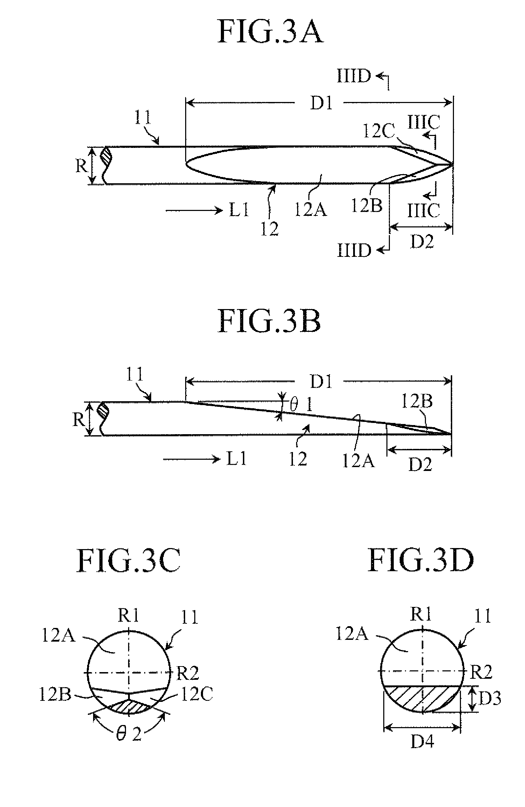

As shown in Fig. 3A to Fig. 3D, the puncture needle 11 has a blade 12 formed at the end. The blade 12 includes a first blade surface 12A, a second blade surface 12B, and a third blade surface 12C. The blade surfaces 12A to 12C are formed by grinding a wire rod and the like. The stainless wire rod having an outer diameter R of 0.2 to 0.4 mm is used as the wire rod for forming the puncture needle 11. The wire rod made of resin, or made of ceramics may also be used.

The first blade surface 12A is, as a whole, arranged at a position evacuated from the needlepoint than the second and the third blade surfaces 12B, 12C, and has the largest occupying area in the blade 12. The first blade surface 12A has a distance D1 in an axial direction L1 to a site most distant from the needlepoint of 1.5 to 2.5 mm, and an intersecting angle θ1 with respect to the axial direction L1 of the puncture needle 11 of 7 to 20 degrees.

The second and the third blade surfaces 12B, 12C define the needlepoint, and are formed so that a distance D2 in the axial direction L1 to a site most distant from the needlepoint is 0.4 to 0.68 mm, and a ratio (D2/D1) of the distance D2 with respect to the distance D1 is in the range of 0.22 to 0.38. The second and the third blade surfaces 12B, 12C are formed so as to be symmetric or substantially symmetric to each other with respect to an axis of symmetry extending along a first radial direction R1 when viewed in the axial direction shown in Fig. 3C, where a sandwiching angle θ2 between the second blade surface 12B and the third blade surface 12B, 12C is set to 120 to 170 degrees, and the like. As shown in Fig. 3D, the cross-section at the site the needlepoint is most distant in the second and the third blade surfaces 12B, 12C has a ratio (D3/D4) of a dimension D3 in the first radial direction R1 and a dimension D4 in the second radial direction R2 orthogonal to the first radial direction R1 set in a range of 0.29 to 0.50, and preferably in a range of 0.33 to 0.50.

The ratio (D1/R) of the distance D1 with respect to the outer diameter (correspond to the outer diameter of the wire rod) R of the portion other than the blade is, for example, 3.75 to 12.50, and the ratio (D2/R) of the distance D2 with respect to the outer diameter R is, for example, 1.0 to 3.5. Furthermore, (D1/D2/R) is set in a range of, for example, 8.5 to 31.3.

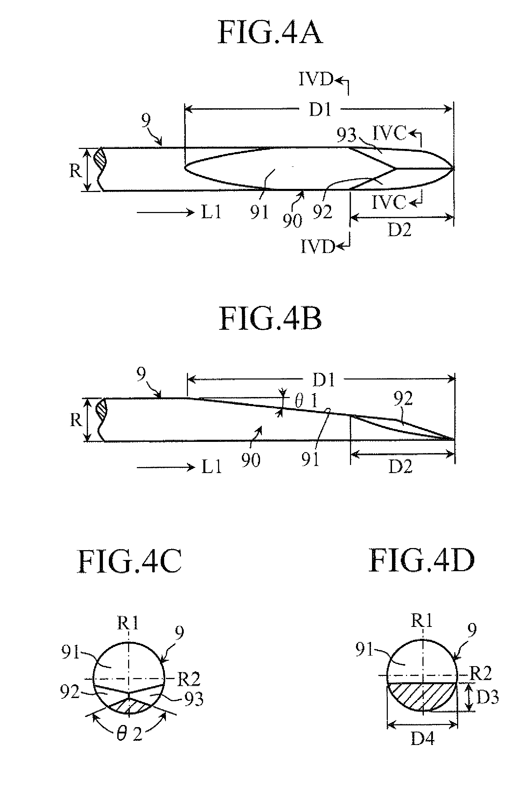

An end of the puncture needle having a general configuration of the prior art is shown in Fig. 4 for comparison. A puncture needle 9 shown in Fig. 4A to Fig. 4D includes a blade 90 with first to third blade surfaces 91, 92, 93, similar to the puncture needle 11 of the present invention described above.

The first blade surface 91 has a distance D1 in the axial direction L1 from the needlepoint to the most distant site of 1.5 to 2.5 mm, and an intersecting angle θ1 with respect to the axial direction L1 of the puncture needle 9 of about 10 degrees.

The second and the third blade surfaces 92, 93 have a distance D2 in the axial direction L1 from the needlepoint to the most distant site set to 0.7 to 1.0 mm. The blade surfaces 92, 93 have a sandwiching angle θ2 when viewed in the axial direction shown in Fig. 4C set to about 140 degrees. The ratio (D1/R) of the distance D 1 with respect to the outer diameter R is, for example, 3.75 to 10, and the ratio (D2/R) of the distance D2 with respect to the outer diameter R is, for example, 1.75 to 4.0. Furthermore, (D1/D2/R) is set to smaller than 8.5.

Compared to the puncture needle 9 shown in Fig. 4A to Fig. 4D, the puncture needle 11 shown in Fig. 3A to Fig. 3D is formed so that the length dimension D2 in the axial direction L1 of the second and the third blade surfaces 12B, 12C is small, the ratio (D1/R) is large, the (D2/R) is small, the (D1/D2/R) is large, and the aspect ratio in the cross-sectional shape is made large (flat) up to the range of the distance D2 from the needlepoint. Thus, the thrust resistance at the portion of the blade 12 formed with the second and the third blade surfaces 12B, 12C becomes small. That is, the boring pain is small up to a constant distance from the needlepoint. Thus, the boring pain can be alleviated by setting the puncture depth to a range of small thrust resistance, and furthermore, even if the puncture depth is set larger than the range of small thrust resistance, the boring pain felt by the person to be taken blood is alleviated since the thrust resistance at the initial stage of insertion is alleviated.

On the other hand, a large incision length in time of insert can be ensured by forming the cross-sectional shape flat in the puncture needle 11. More specifically, if the puncture needles 9, 11 shown in Fig. 3 and Fig. 4 are used, the incision will have a circular arc shape in correspondence to the shape of the circular arc portion of the blade 12. Thus, an incision length sufficient to ensure the desired bleeding amount can be ensured even if the puncture depth is small by providing a flat cross-sectional shape.

In the puncture needle 11, the wire rod having a small outer diameter R does not need to be actively used for the wire rod for forming the puncture needle 11 since the bleeding amount can be appropriately ensured while alleviating the boring pain. In other words, even if the puncture needle 11 is formed from a relatively thick wire rod, the puncture needle 11 alleviates the boring pain, and ensures sufficient bleeding amount even if the puncture depth is small. Thus, the machining is facilitated when forming the first to the third blade surfaces 12A to 12C by grinding etc., and furthermore, defects such as bend, chip, and burr can be reduced while benefiting from the above advantages by forming the puncture needle 11 with the relatively thick wire rod.

The present invention obviously can be changed in various ways, and for example, the shape of the body 10 of the lancet 1 is not limited to a cylindrical shape, and may be of any shape as long it can be held by the lancet holder 20 of the lancing device 2, and furthermore, the lancet of the present invention is applicable to those used in lancing devices other than the lancing device 2 having the configuration shown in Fig. 2. The lancet of the present invention may be integrated with an analyzing tool such as a biosensor.

### Example 1

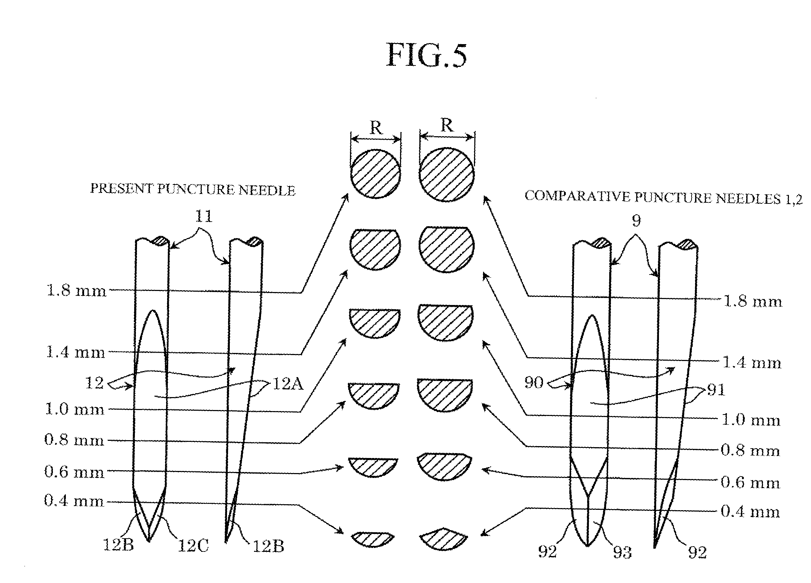

In the present example, the relationship of the circular arc length L of the blade and the cross-sectional area A of the blade was reviewed for a plurality of puncture needles. The dimension, the angle, and the like of each part in each puncture needle are as shown in the table 1 below. The reference numerals in table 1 correspond to the dimension, the angle, and the like shown in Fig. 3 and Fig. 4. L in table 1 is the circular arc length (see Fig. 5) of the cross-section in the radial direction of the blade, A is a cross-sectional area at the cross-section, and the numbers from 0.2 to 1.0 in the items of the circular arc length L and the cross-sectional area A show the distance (mm) from the needlepoint.

**Table 1**

| No. | | 1 | 2 | 3 | 4 | 5 | 6 | 7 | 8 | 9 | 10 |
|---|---|---|---|---|---|---|---|---|---|---|---|
| R (mm) | | 0.2 | 0.2 | 0.31 | 0.31 | 0. 345 | 0.345 | 0.345 | 0. 38 | 0.38 | 0.4 |
| D1 (mm) | | 1.5 | 1 | 1.7 | 1.5 | 1.8 | 1.7 | 1.6 | 1. 90 | 1.7 | 1.95 |
| D2 (mm) | | 0.4 | 0.6 | 0.5 | 0.7 | 0.5 | 0. 4 | 0.7 | 0. 45 | 0.7 | 0.5 |
| *θ*1(° ) | | 7 | 10 | 10 | 10 | 10 | 11 | 10 | 10 | 10 | 10 0 |
| *θ*2(° ) | | 120 | 140 | 140 | 140 | 140 | 170 | 140 | 140 | 140 | 140 |
| L (mm) | 0.2 | 0.138 | 0.157 | 0.189 | 0.198 | 0.217 | 0.249 | 0.220 | 0.255 | 0.241 | 0.262 |
| | 0.4 | 0.243 | 0.262 | 0.302 | 0.329 | 0.360 | 0.375 | 0.364 | 0.423 | 0.400 | 0.435 |
| | 0.6 | 0.293 | 0.374 | 0.408 | 0.451 | 0.463 | 0.459 | 0.499 | 0.518 | 0.547 | 0.552 |
| | 0.8 | 0.342 | 0.455 | 0.480 | 0.551 | 0.534 | 0.537 | 0. 605 | 0.589 | 0.660 | 0. 623 |
| | 1 | 0.393 | 0.628 | 0.550 | 0.625 | 0.605 | 0.615 | 0.679 | 0.660 | 0.733 | 0.694 |
| A (mm²) | 0.2 | 0.004 | 0.005 | 0.006 | 0.007 | 0.008 | 0.008 | 0.009 | 0.012 | 0.010 | 0.013 |
| | 0.4 | 0.009 | 0.013 | 0.015 | 0.021 | 0.021 | 0.020 | 0.027 | 0.029 | 0.032 | 0.033 |
| | 0.6 | 0.014 | 0.022 | 0.026 | 0.036 | 0.033 | 0.033 | 0.045 | 0.042 | 0.054 | 0.048 |
| | 0.8 | 0.019 | 0.028 | 0.037 | 0.047 | 0.045 | 0.046 | 0.058 | 0.055 | 0.069 | 0.062 |
| | 1 | 0.023 | 0.031 | 0.047 | 0.058 | 0.058 | 0.059 | 0.069 | 0.069 | 0.081 | 0.076 |
| D1/R | | 7.50 | 5.00 | 5.48 | 4.84 | 5.22 | 4.93 | 4.64 | 5.00 | 4.47 | 4.88 |
| D2/R | | 2.00 | 3.00 | 1.61 | 2. 26 | 1.45 | 1.16 | 2.03 | 1.18 | 1.84 | 1.25 |
| D2/D1 | | 0.27 | 0.60 | 0.29 | 0.47 | 0.28 | 0.24 | 0.44 | 0.24 | 0.41 | 0.26 |
| D3/D4 | | 0.32 | 0.68 | 0.34 | 0.55 | 0.36 | 0.29 | 0.56 | 0.33 | 0.53 | 0.37 |
| D1/D2/R | | 18.75 | 8.33 | 10.97 | 6.91 | 10.43 | 12.32 | 6.63 | 11.11 | 6.39 | 9.75 |

If the skin is punctured using the puncture needle, the incision will have a circular arc shape in correspondence to the shape of the circular arc portion of the blade of the puncture needle. Thus, a large incision length can be ensured if the length of the circular arc portion (circular arc length) L of the blade is large. The bleeding amount in puncture depends on the incision length. Thus, a large bleeding amount can be ensured by ensuring a circular arc length L.

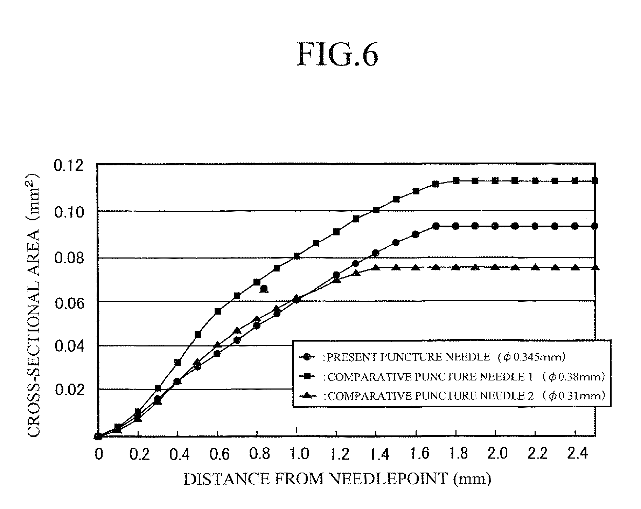

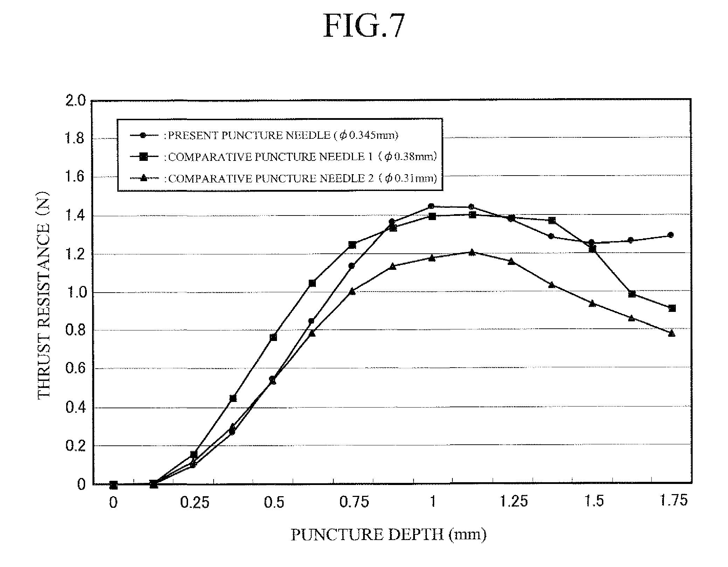

The cross-sectional area A of the portion to be inserted in the skin is correlated with the thrust resistance. That is, the thrust resistance becomes large if the cross-sectional area A is large, and the thrust resistance becomes small if the cross-sectional area A is small. This is also apparent from the results obtained in examples 2 and 3 to be hereinafter described. That is, as apparent with reference to Fig. 6 and Fig. 7, the thrust resistance becomes larger the larger the cross-sectional area up to a constant puncture depth (distance D2 up to the portion of second and third blade surfaces). The thrust resistance is also correlated with the boring pain of when puncturing the skin. That is, the boring pain felt by the person to be taken blood becomes greater the larger the thrust resistance, and the boring pain felt by the person to be taken blood becomes smaller the smaller the thrust resistance. Thus, the cross-sectional area A of the portion to be inserted in the skin of the puncture needle influences the boring pain felt by the person to be taken blood, where the boring pain felt by the person to be taken blood is smaller the smaller the cross-sectional area A and thus is preferable.

Therefore, preferably, the circular arc length L is large and the cross-sectional area A is small from the standpoint of reducing the boring pain while sufficiently ensuring the bleeding amount. It is not preferable to have the circular arc length L (incision length) longer than necessary as long as sufficient bleeding amount can be ensured, and it merely needs to be greater than or equal to a constant value. For instance, to ensure a bleeding amount of about 3.0 µL, the circular arc length L is greater than or equal to 0.94 mm if the puncture depth is set to 1.2 mm; and to ensure a bleeding amount of about 1.5 µL, the circular arc length L is greater than or equal to 0.61 mm if the puncture depth is set to 1.0 mm.

Reviewing table 1 in view of the above, comparing puncture needles No. 1 and No. 2 having an outer diameter R of 0.2 mm, the puncture needle of No. 1 has a smaller cross-sectional area A than the puncture needle of No. 2. The puncture needle of No. 1 has a slightly smaller circular arc length L than the puncture needle of No. 2, but has a sufficient circular arc length L to ensure sufficient bleeding amount. Therefore, comparing the puncture needles No. 1 and No. 2 having the outer diameter R of 0.2 mm, the puncture needle No. 1 having smaller cross-sectional area A and less boring pain is superior.

Comparing puncture needles No. 3 and No. 4 having an outer diameter R of 0.31 mm, the puncture needle of No. 3 has a smaller cross-sectional area A than the puncture needle of No. 4. The puncture needle of No. 3 has a slightly smaller circular arc length L than the puncture needle of No. 4, but has a sufficient circular arc length L to ensure sufficient bleeding amount. Therefore, comparing the puncture needles No. 3 and No. 4 having the outer diameter R of 0.31 mm, the puncture needle No. 3 having smaller cross-sectional area A and less boring pain is superior.

Comparing puncture needles No. 5, No. 6 and No. 7 having an outer diameter R of 0.345 mm, the puncture needles of No. 5 and No. 6 have a smaller cross-sectional area A than the puncture needle of No. 7. The puncture needle of No. 5 has substantially the same circular arc length L as the puncture needle of No. 7. The puncture needle of No. 6 has a longer circular arc length L than the puncture needle of No. 7. Therefore, comparing the puncture needles No. 5 to No. 7 having the outer diameter R of 0.345 mm, the puncture needles No. 5 and No. 6 are preferable, and the puncture needle of No. 5 is the most preferable.

Comparing puncture needles No. 8 and No. 9 having an outer diameter R of 0.38 mm, the puncture needle of No. 8 has a smaller cross-sectional area A than the puncture needle of No. 9 other than when the distance from the needlepoint is 0.2 mm. The puncture needle of No. 8 has a smaller circular arc length L than the puncture needle of No. 9 when the distance from the needlepoint exceeds 0.6 mm, but has a sufficient circular arc length L to ensure sufficient bleeding amount. Therefore, comparing the puncture needles No. 8 and No. 9 having the outer diameter R of 0.38 mm, the puncture needle No. 8 having smaller cross-sectional area A and less boring pain is superior.

The puncture needle No. 10 having an outer diameter R of 0.40 mm will be reviewed. The outer diameter R of the puncture needle No. 10 is 0.40 mm and is not preferable since the boring pain becomes too great to be used as the puncture needle. The puncture needle No. 10 has the same cross-sectional area A until the distance from the needlepoint is 0.4 mm and has a smaller cross-sectional area A when the distance from the needlepoint becomes larger than 0.6 mm, compared to the puncture needle No. 9 having an outer diameter R of 0.38 mm. The puncture needle No. 10 obviously has a long circular arc length L since the outer diameter R is large. Therefore, the puncture needle No. 10 having an outer diameter R of 0.40 mm is superior over the puncture needle No. 9 having an outer diameter R of 0.38 mm.

From the above review, the puncture needles No. 1, No. 3, No. 5, No. 6, No. 8, and No. 10 are preferable from the standpoint of alleviating the boring pain while ensuring sufficient bleeding amount. The parameters for distinguishing the puncture needles No. 1, No. 3, No. 5, No. 6, No. 8, and No. 10 and the puncture needles No. 2, No. 4, No. 7, and No. 9 were considered, and D1/R, D2/R, D2/D1, D3/D4, and D1/D2/R shown in table 1 were derived.

As apparent from table 1, D1/R is preferably in the range of 3.75 to 12.5, D2/R is preferably in the range of 1.0 to 3.5, D2/D1 is preferably in the range of 0.22 to 0.38, D3/R4 is preferably in the range of 0.33 to 0.50, and D1/D2/R is preferably in the range of 7.0 to 31.3

The puncture needles No. 1 to No. 9 evaluated here have R in the range of 0,2 to 4.0 mm, D1 in the range of 1.5 to 2.5 mm, D2 in the range of 0.4 to 0.68 mm, θ1 in the range of 7 to 20°, and θ2 in the range of 120 to 170°.

### Example 2

In the present example, change in cross-sectional area of the end was reviewed for the present puncture needle and the comparative puncture needles 1 and 2 shown in the table 2 below. The present puncture needle is the puncture needle No. 5 in table 1, and the comparative puncture needles 1 and 2 are puncture needles No. 9 and No. 4, respectively, in table 1. The reference numerals in table 2 correspond to the dimension, the angle, and the like shown in Fig. 3 and Fig. 4.

**Table 2**

| | PRESENT PUNCTURE NEEDLE | COMPARATIVE PUNCTURE NEEDLE 1 | COMPARATIVE PUNCTURE NEEDLE 2 |
|---|---|---|---|
| R | 0. 345mm | 0.38mm | 0.31mm |
| D1 | 1.8mm | 1. 7mm | 1. 5mm |
| D 2 | 0. 5mm | 0. 7mm | 0.7mm |
| D1/R | 5.22 | 4.47 | 4.84 |
| D 2 / R | 1.45 | 1. 84 | 2. 26 |
| D1/D2/R | 10.43 | 6.39 | 6.91 |
| D2/D1 | 0.28 | 0.41 | 0.47 |
| D3/D4 | 0.36 | 0.53 | 0.55 |
| θ1 | 10 DEGREES | 10 DEGREES | 10 DEGREES |
| θ2 | 140 DEGREES | 140 DEGREES | 140 DEGREES |

The cross-sectional shape of each puncture needle is more or less as shown in Fig. 5, and the calculation result from the simulation of the actual cross-sectional area is as shown in Fig. 6.

As apparent from Fig. 6, in the present puncture needle, the cross sectional area is the same as the comparative puncture needle 2 having an outer diameter R smaller than the present puncture needle, and the cross-sectional area is significantly smaller than the comparative puncture needle 1 having an outer diameter R larger than the present puncture needle in the range of 1.2 mm from the needlepoint. Thus, the thrust resistance of the same extent as the comparative puncture needle 2 having an outer diameter R smaller than the present puncture needle is expected, and the boring pain is assumed to be small up to a constant range from the needlepoint.

### Example 3

In the present example, the relationship between the puncture depth and the thrust resistance was reviewed for the puncture needles (present puncture needle, comparative puncture needles 1, 2) having the configuration shown in table 1.

The thrust resistance was measured using push-pull gauge FGX-0.2R (manufactured by NIDEC-Shimpo Co.). The puncture needle was fixed with respect to the push-pull gauge using a pin chuck 4PC-05 (manufactured by NIDEC-Shimpo Co.) for a needle fixing jig, and the projecting amount of the puncture needle was set to 10 mm. The material cut to 25 × 25 mm from polypropylene having a thickness of 2 mm (PPS-C 6T (manufactured by Sheedom Com.) was used as the piercing material. The piercing material was fixed to a fixing jig using four screws. A tightening torque of the screw was set to 2.0kgf·cm. The thrust speed was 20cm/sec, and the thrust resistance was measured every 125msec. The measurement result of the thrust resistance is shown in Fig. 7.

As apparent from Fig. 7, the thrust resistance is the same as the comparative puncture needle 2 having an outer diameter R smaller than the present puncture needle up to the range of 0.6 mm from the needlepoint. That is, the present puncture needle has a small thrust resistance up to a constant range from the needlepoint, and thus the boring pain becomes the same extent although the outer diameter R is larger than the comparative puncture needle 2.

### Example 4

In the present example, in the present example, the bleeding amount of when the puncture depth is 1 mm was reviewed for the lancet (present lancet, comparative lancet) including the present puncture needle or the comparative puncture needle 1 having the configuration shown in table 2. The comparative lancet uses the comparative puncture needle 1 which outer diameter R is larger than the present puncture needle of the present lancet, where greater bleeding amount can be ensured than the present lancet when focusing on the outer diameter R.

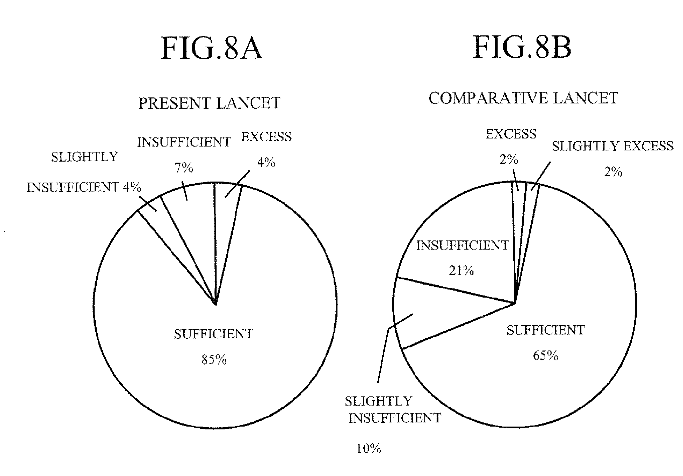

The bleeding amount was checked by a monitor test on whether or not the necessary bleeding amount was obtained in one puncture. More specifically, the present lancet and the comparative lancet were distributed to fifty-six monitors, the respective lancet was attached to the lancing device used by the monitor, and the bleeding amount of when the puncture depth is set to 1 mm was evaluated in five levels of "excess", "slightly excess", "sufficient", "slightly insufficient", and "insufficient". The result of the monitor test is shown in Fig. 8.

As apparent from Fig. 8, the preset lancet is obtained a result that sufficient amount of blood is obtained compared to the comparative lancet. That is, the present lancet was found to obtain sufficient amount of blood even if the puncture depth is relatively shallow of about 1 mm.

According to the above examples 1 to 4, it can be said that the puncture needle and the lancet according to the present invention have small thrust resistance, small boring pain, and can collect sufficient amount of blood. Such effects are obtained by devising the formation modes of the first to the third blade surfaces without unreasonably increasing the outer diameter of the puncture needle or unreasonably decreasing the outer diameter of the puncture needle.

## Claims

1. A puncture needle having a blade arranged at an end; wherein
the blade includes a first blade surface, a second blade surface, and a third blade surface;
the first blade surface is arranged at a position evacuated from a needlepoint than the second and the third blade surfaces as a whole, and has a site most distant from the needlepoint formed within a range of 1.5 to 2.5 mm as a distance D1 in an axial direction from the needlepoint; and
the second and third blade surfaces define the needlepoint, and a site most distant from the needlepoint is formed within a range of 0.4 to 0.68 mm as a distance D2 in the axial direction from the needlepoint.

2. The puncture needle according to claim 1, wherein a ratio (D2/D1) of the distance D2 with respect to the distance D1 is set in a range of 0.22 to 0.38.

3. The puncture needle according to claim 1, wherein (D1/D2/R) is set in a range of 8.5 to 31.3 where R is an outer diameter of a portion other than the blade.

4. The puncture needle according to claim 1, wherein a cross-section at the site most distant from the needlepoint of the second and the third blade surfaces has a ratio (D3/D4) of a dimension D3 in a first radial direction along a normal line of the first blade surface and a dimension D4 in a second radial direction orthogonal to the first radial direction set in a range of 0.29 to 0.50.

5. The puncture needle according to claim 4, wherein the ratio (D3/D4) is set in a range of 0.33 to 0.50.

6. The puncture needle according to claim 1, wherein the first blade surface has an intersecting angle θ1 with respect to the axial direction of the puncture needle of 7 to 20 degrees.

7. The puncture needle according to claim 1, wherein the second and the third blade surfaces are formed symmetric or substantially symmetric to each other with respect to an axis of symmetry extending along a normal line of the first blade surface when viewed in the axial direction.

8. The puncture needle according to claim 7, wherein a sandwiching angle θ2 between the second blade surface and the third blade surface is 120 to 170 degrees.

9. The puncture needle according to claim 1, wherein an outer diameter R of the portion other than the blade is 0.2 to 0.4 mm.

10. The puncture needle according to claim 1, wherein a ratio (D1/R) of the distance D1 in the axial direction from the needlepoint of the site most distant from the needlepoint in the first blade surface with respect to the outer diameter R of the portion other than the blade is 3.75 to 12.50, and a ratio (D2/R) of the distance D2 in the axial direction from the needlepoint of the site most distant from the needlepoint in the second and the third blade surfaces with respect to the outer diameter R is 1.0 to 3.5.

11. A puncture needle having a blade arranged at an end; wherein
the blade includes a first blade surface, a second blade surface, and a third blade surface;
the first blade surface is arranged at a position evacuated from a needlepoint than the second and the third blade surfaces as a whole;
the second and third blade surfaces define the needlepoint; and a ratio (D2/D1) of a distance D2 in an axial direction from the needlepoint of a site most distant from the needlepoint in the second and the third blade surfaces with respect to a distance D1 in the axial direction from the needlepoint of a site most distant from the needlepoint in the first blade surface is set in a range of 0.22 to 0.38.

12. The puncture needle according to claim 11, wherein (D1/D2/R) is set in a range of 8.5 to 31.3 where R is an outer diameter of a portion other than the blade.

13. The puncture needle according to claim 11, wherein a cross-section at the site most distant from the needlepoint of the second and the third blade surfaces has a ratio (D3/D4) of a dimension D3 in a first radial direction along a normal line of the first blade surface and a dimension D4 in a second radial direction orthogonal to the first radial direction set in a range of 0.29 to 0.50.

14. The puncture needle according to claim 13, wherein the ratio (D3/D4) is set in a range of 0.33 to 0.50.

15. The puncture needle according to claim 11, wherein the first blade surface has an intersecting angle θ1 with respect to the axial direction of the puncture needle of 7 to 20 degrees.

16. The puncture needle according to claim 11, wherein the second and the third blade surfaces are formed symmetric or substantially symmetric to each other with respect to an axis of symmetry along a normal line of the first blade surface when viewed in the axial direction.

17. The puncture needle according to claim 16, wherein a sandwiching angle θ2 between the second blade surface and the third blade surface is 120 to 170 degrees.

18. The puncture needle according to claim 11, wherein an outer diameter R of the portion other than the blade is 0.2 to 0.4 mm.

19. The puncture needle according to claim 11, wherein a ratio (D1/R) of the distance D 1 in the axial direction from the needlepoint of the site most distant from the needlepoint at the first blade surface with respect to the outer diameter R of the portion other than the blade is 3.75 to 12.50, and a ratio (D2/R) of the distance D2 in the axial direction from the needlepoint of the site most distant from the needlepoint at the second and the third blade surfaces with respect to the outer diameter R is 1.0 to 3.5.

20. A puncture needle having a blade arranged at an end; wherein
the blade includes a first blade surface, a second blade surface, and a third blade surface;
the first blade surface is arranged at a position evacuated from a needlepoint than the second and the third blade surfaces as a whole;
the second and third blade surfaces define the needlepoint; and
(D1/D2/R) is set in a range of 8.5 to 31.3 where D1 is a distance in an axial direction from the needlepoint of a site most distant from the needlepoint in the first blade surface, D2 is a distance in the axial direction from the needlepoint of a site most distant from the needlepoint in the second and the third blade surfaces, and R is an outer diameter of a portion other than the blade.

21. The puncture needle according to claim 20, wherein a cross-section at the site most distant from the needlepoint of the second and the third blade surfaces has a ratio (D3/D4) of a dimension D3 in a first radial direction along a normal line of the first blade surface and a dimension D4 in a second radial direction orthogonal to the first radial direction set in a range of 0.29 to 0.50.

22. The puncture needle according to claim 21, wherein the ratio (D3/D4) is set in a range of 0.33 to 0.50.

23. The puncture needle according to claim 20, wherein the first blade surface has an intersecting angle θ1 with respect to the axial direction of the puncture needle of 7 to 20 degrees.

24. The puncture needle according to claim 20, wherein the second and the third blade surfaces are formed symmetric or substantially symmetric to each other with respect to an axis of symmetry along a normal line of the first blade surface when viewed in the axial direction.

25. The puncture needle according to claim 24, wherein a sandwiching angle θ2 between the second blade surface and the third blade surface is between 120 to 170 degrees.

26. The puncture needle according to claim 20, wherein an outer diameter R of the portion other than the blade is 0.2 to 0.4 mm.

27. The puncture needle according to claim 20, wherein a ratio (D1/R) of the distance D1 in the axial direction from the needlepoint of the site most distant from the needlepoint in the first blade surface with respect to the outer diameter R of the portion other than the blade is 3.75 to 12.50, and a ratio (D2/R) of the distance D2 in the axial direction from the needlepoint of the site most distant from the needlepoint in the second and the third blade surfaces with respect to the outer diameter R is 1.0 to 3.5.

28. A puncture needle having a blade arranged at an end; wherein
the blade includes a first blade surface, a second blade surface, and a third blade surface;
the first blade surface is arranged at a position evacuated from a needlepoint than the second and the third blade surfaces as a whole;
the second and third blade surfaces define the needlepoint; and a cross-section at a site most distant from the needlepoint in the second and
the third blade surfaces has a ratio (D3/D4) of a dimension D3 in a first radial direction along a normal line of the first blade surface and a dimension D4 in a second radial direction orthogonal to the first radial direction set in a range of 0.29 to 0.50.

29. The puncture needle according to claim 28, wherein the ratio (D3/D4) is set in a range of 0.33 to 0.50.

30. The puncture needle according to claim 28, wherein the first blade surface has an intersecting angle θ1 with respect to the axial direction of the puncture needle of 7 to 20 degrees.

31. The puncture needle according to claim 28, wherein the second and the third blade surfaces are formed symmetric or substantially symmetric to each other with respect to an axis of symmetry along a normal line of the first blade surface when viewed in the axial direction.

32. The puncture needle according to claim 31, wherein a sandwiching angle θ2 between the second blade surface and the third blade surface is 120 to 170 degrees.

33. The puncture needle according to claim 28, wherein an outer diameter R of the portion other than the blade is 0.2 to 0.4 mm.

34. The puncture needle according to claim 28, wherein a ratio (D1/R) of the distance D1 in the axial direction from the needlepoint of the site most distant from the needlepoint in the first blade surface with respect to the outer diameter R of the portion other than the blade is 3.75 to 12.50, and a ratio (D2/R) of the distance D2 in the axial direction from the needlepoint of the site most distant from the needlepoint in the second and the third bade surfaces with respect to the outer diameter R is 1.0 to 3.5.

35. A lancet comprising a puncture needle, and a body for holding the puncture needle, wherein the puncture needle is as described in any one of claims 1 to 34.
